(19) Europäisches Patentamt
European Patent Office
Office européen des brevets

(11) Veröffentlichungsnummer: **0 086 350**
**A2**

(12) # EUROPÄISCHE PATENTANMELDUNG

(21) Anmeldenummer: **83100508.7**

(22) Anmeldetag: **21.01.83**

(51) Int. Cl.³: **C 07 C 172/00,** C 07 J 7/00, C 07 J 9/00, C 07 J 17/00, C 07 J 51/00, C 07 D 317/20

(30) Priorität: **12.02.82 US 348388**

(43) Veröffentlichungstag der Anmeldung: **24.08.83 Patentblatt 83/34**

(84) Benannte Vertragsstaaten: **AT BE CH DE FR GB IT LI NL**

(71) Anmelder: **F. HOFFMANN-LA ROCHE & CO. Aktiengesellschaft, CH-4002 Basel (CH)**

(72) Erfinder: **Partridge, John Joseph, 121 Norwood Avenue, Upper Montclair, N.J. (US)**
Erfinder: **Shiuey, Shian-Jan, 331 Bloomfield Avenue, Nutley, N.J. (US)**
Erfinder: **Uskokovic, Milan Radoje, 253 Highland Avenue, Upper Montclair, N.J. (US)**

(74) Vertreter: **Mahé, Jean et al, Postfach 3255 Grenzacherstrasse 124, CH-4002 Basel (CH)**

(54) **Verfahren zur Herstellung von Cholesterinderivaten.**

(57) Verfahren zur Herstellung des 1α,25,26-Trihydroxycholecalciferols in Form des C–25 R- oder S-Epimeren oder in Form eines Gemisches davon, ausgehend von einer Verbindung der Formel

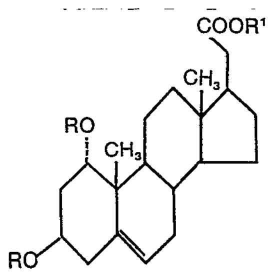

I

worin R¹ Niederalkyl, Phenyl oder substituiertes Phenyl, R eine Gruppe der Formel

$$-C(R^2,R^3,OR^4)$$

$R^2$ Wasserstoff oder Niederalkyl und $R^3$ und $R^4$ Niederalkyl oder zusammen Niederalkylen sind, und in diesem Verfahren vorkommende neue Zwischenprodukte.

EP 0 086 350 A2

F.Hoffmann-La Roche & Co. Aktiengesellschaft,Basel,Schweiz

RAN 4212/33

# Verfahren zur Herstellung von Cholesterinderivaten

Die Erfindung betrifft ein neues Verfahren zur Herstellung des 1α,25,26-Trihydroxycholecalciferols in Form des C-25 R- oder S-Epimeren oder in Form von Gemischen davon sowie neue in diesem Verfahren vorkommende Zwischenprodukte.

Im Rahmen der Erfindung bezieht sich der Ausdruck "nieder" auf Gruppen mit 1-8 C-Atomen. Die Niederalkylgruppen können geradkettig oder verzweigt sein. Beispiele davon sind Methyl, Aethyl, n-Propyl, i-Propyl, t-Butyl, Hexyl, Heptyl und Octyl. Beispiele von Niederalkylengruppen sind Methylen, Aethylen, Propylen, Butylen, Amylen, Hexylen, Heptylen und Octylen. Beispiele von Niederalkoxygruppen sind Methoxy, Aethoxy, Isopropoxy und t-Butoxy. Beispiele von Niederalkanoylgruppen sind Formyl, Acetyl, Butyryl und Hexanoyl. Der Ausdruck "substituiertes Phenyl" betrifft Phenyl, das durch einen oder mehreren Halogenatomen Fluor, Chlor, Brom oder Jod, oder durch einer oder mehreren der Gruppen Nitro, Cyan oder Trifluormethyl substituiert ist.

Mé/ 18.1.83

Das erfindungsgemässe Verfahren ist dadurch gekennzeichnet, dass man

a) eine Verbindung der Formel

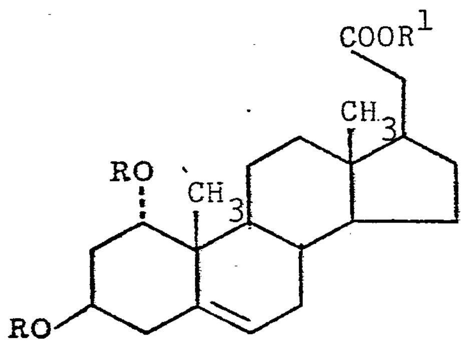

I

worin $R^1$ Niederalkyl, Phenyl oder substituiertes Phenyl und R eine Gruppe der Formel

$$-C(R^2,R^3,OR^4)$$

$R^2$ Wasserstoff oder Niederalkyl und $R^3$ und $R^4$ Niederalkyl oder zusammen $C_{3-6}$-Alkylen sind,
mit einem Lithium-, Natrium-, Kalium-, Magnesium- oder Zinkorganometallischen Reagenzien umsetzt,

b) das Produkt der Stufe a) der Formel

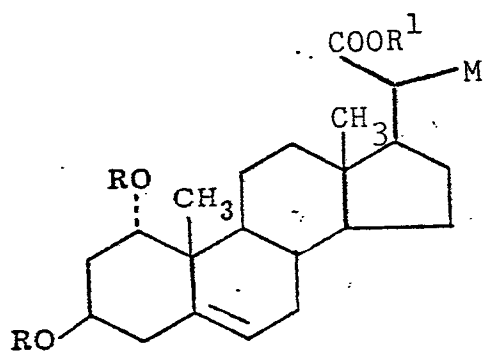

II

worin R und $R^1$ die obige Bedeutung haben und M Lithium, Natrium, Kalium, Magnesium/2 oder Zink/2 sind,
mit einer Verbindung der Formel

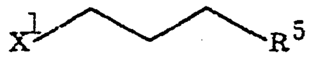

III

worin $X^1$ Jod, Brom, Chlor, Niederalkylsulfonyloxy, Phenylsulfonyloxy oder substituiertes Phenylsulfonyloxy und $R^5$ eine Gruppe der Formel

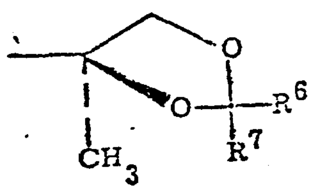

oder

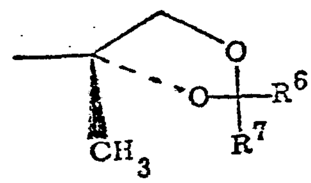

$R^6$ und $R^7$ Niederalkyl oder zusammen Niederalkylen sind,

c) den Ester von Stufe b) der Formel

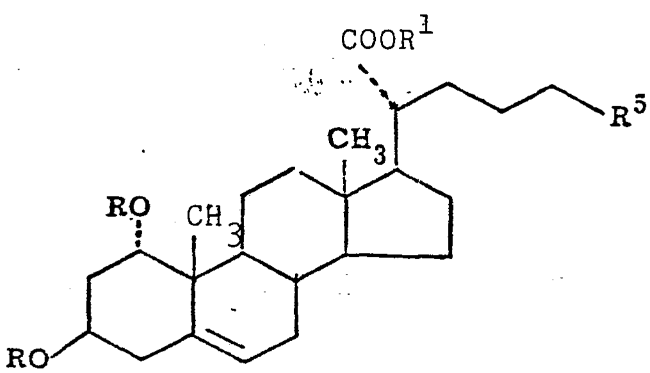

IV

worin R, $R^1$ und $R^5$ die obige Bedeutung haben, reduziert,

d) im Produkt der Stufe c) und der Formel

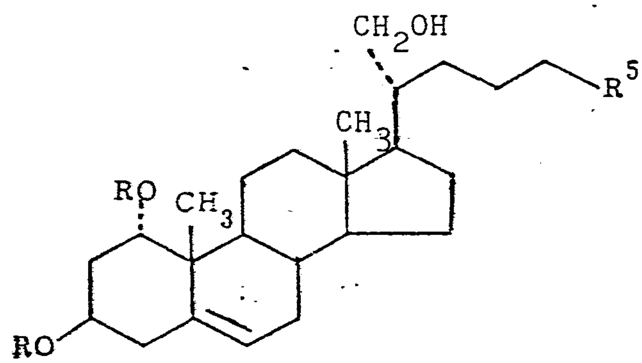

V

worin R und $R^5$ die obige Bedeutung haben, die Hydroxygruppe durch eine Gruppe $X^1$, worin $X^1$ obige Bedeutung hat, ersetzt,

e) das Produkt von Stufe d) und der Formel

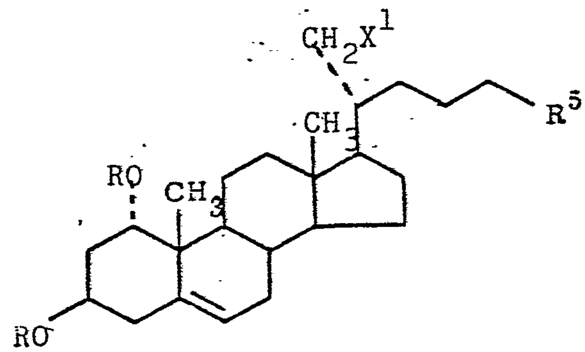

VI

worin R, $R^5$ und $X^1$ die obige Bedeutung haben, mit einem Reduktionsmittel umsetzt,

f) das Produkt der Stufe e) und der Formel

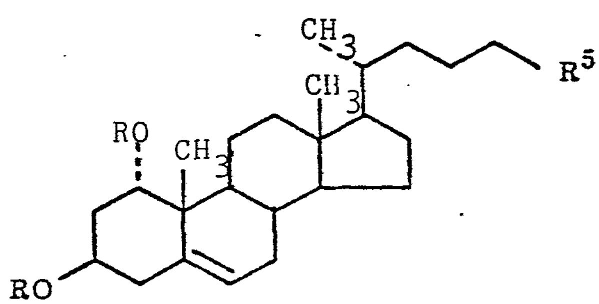

VII-A

worin R und $R^5$ die obige Bedeutung haben, mit einer starken Säure in einem Gemisch eines protischen Lösungsmittels und eines Ketallösungsmittels umsetzt,

g) das Produkt der Stufe f) und der Formel

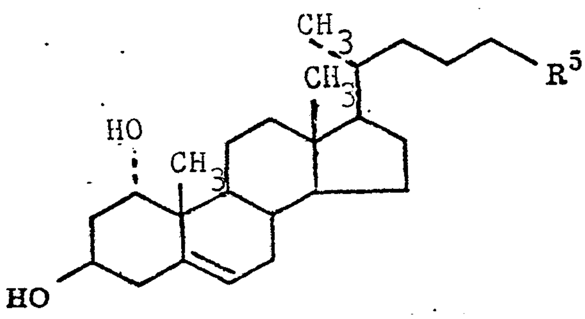

VII-B

worin $R^5$ obige Bedeutung hat, mit einem Niederalkanoylierungsmittel umsetzt,

h) das Produkt der Stufe g) und der Formel

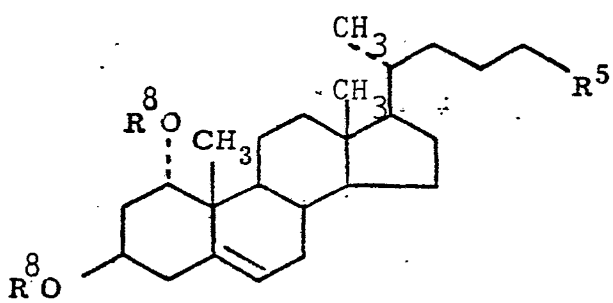

VII-C

worin $R^5$ die obige Bedeutung hat und $R^8$ Nieder-alkanoyl ist, in 7-Stellung allylisch halogeniert,

i) das Produkt der Stufe h) und der Formel

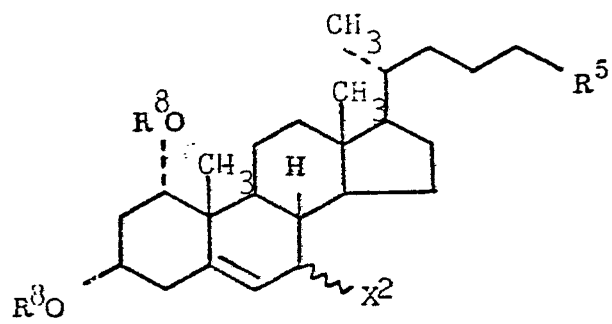

VIII

worin $R^5$ und $R^8$ die obige Bedeutung haben und $X^2$ Brom, Chlor oder Jod ist,
dehydrohalogeniert,

j) das Produkt der Stufe i) und der Formel

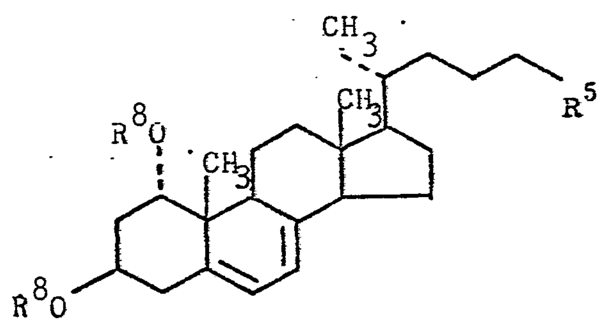

IX

worin $R^5$ und $R^8$ obige Bedeutung haben,
bestrahlt,

k) das Produkt der Stufe j) und der Formel

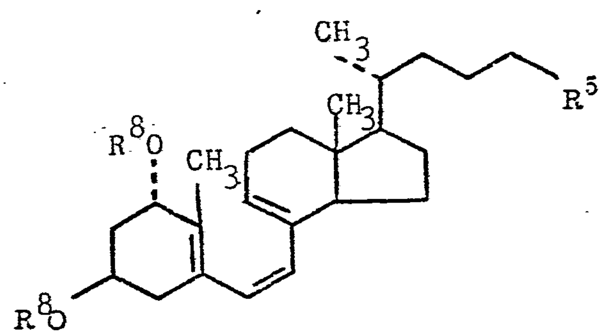

X-A

worin $R^5$ und $R^8$ obige Bedeutung haben, verseift,

l) das Produkt der Stufe k) und der Formel

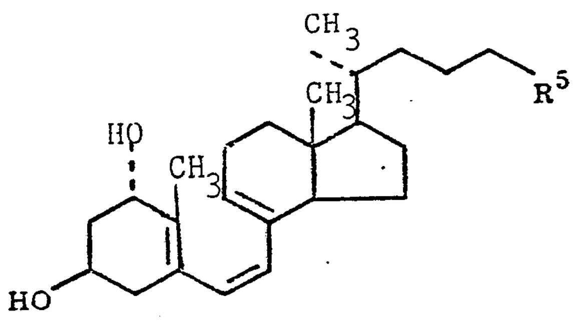

X-B

worin $R^5$ obige Bedeutung hat, thermisch isomerisiert und

m) das Produkt der Stufe l) und der Formel

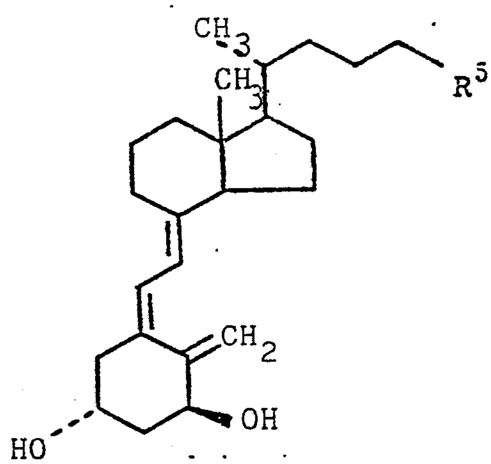

XI

worin $R^5$ obige Bedeutung hat, mit einer Säure in einem protischen Lösungsmittel behandelt.

Die Ausgangsmaterialien der Formel I können ausgehend von 1α,3β-Dihydroxyandrost-5-en-17-on (J.A.C.S. 82, 1960, 4026) in an sich bekannter Weise hergestellt werden. Zum Beispiel kann die Herstellung des 1α,3β-Bis-(tetrahydro-2H-pyran-2-yl-oxy)-pregn-5-en-21-carbonsäureäthylesters via

1α,3β-Bis-(tetrahydro-2H-pyran-2-yl-oxy)androst-5-en-17-on und [1α,3β,17(20)E]-1,3-Bis-(tetrahydro-2H-pyran-2-yl-oxy)-pregna-5,17(20)-dien-21-carbonsäureäthylester nach der in J. Chem. Soc. Perkin Trans I, 1978, 1282 beschriebenen Methode durchgeführt werden.

Als organometallische Reagenzien in Stufe a) des erfindungsgemässen Verfahrens können z.B. Lithiumdiisopropylamid, Natriumhexamethyldisilazan oder Kaliumhydrid verwendet werden.

Die Umsetzung eines metallierten Esters der Formel II mit einer Verbindung der Formel III in R- oder S-Form oder in Form eines Gemisches davon, wird vorzugsweise in situ durchgeführt.

Die Verbindungen der Formel III können nach der in J.A.C.S. 103, 1981, 1253 beschriebenen Methode hergestellt werden.

Die Alkylierungsstufe b) kann bei einer Temperatur zwischen -78 und +60°C, vorzugsweise zwischen -40 und 0°C, durchgeführt werden.

Die Stufen a) und b) können in einem inerten aprotischen Lösungsmittel, wie einem Aether, z.B. Diäthyläther, Tetrahydrofuran, Dimethoxyäthan oder Dioxan; einem Amid, z.B. Hexamethylphosphoramid; vorzugsweise in letzterem oder in Tetrahydrofuran, insbesondere in Gemischen davon, durchgeführt werden.

Die Verbindungen der Formeln IV, V, VI, VII-A, VII-B, VII-C, VIII, IX, X-A, X-B und XI, die eine Gruppe $R^5$ enthalten, können in Form des C-25 R- oder S-Epimeren oder in Form von Gemischen davon vorliegen.

In der Reduktionsstufe c) werden Komplexe Metallhydridreduktionsmittel, wie Alkalimetallaluminiumhydride,

z.B. Lithiumaluminiumhydrid; Mono-, Di- oder Tri-(niederalkoxy)-alkalimetallaluminiumhydride, z.B. Lithium-tri(t-butoxy)-aluminiumhydrid; Mono-, Di- oder Tri-(niederalkoxy-niederalkoxyalkalimetallaluminiumhydride, z.B. Natrium-bis-(2-methoxyäthoxy)-aluminiumhydrid; oder Di-(niederalkyl)-aluminiumhydride, z.B. Di-(isobutyl)-aluminiumhydrid verwendet. Ein bevorzugtes Reduktionsmittel ist Lithiumaluminiumhydrid. Bei dieser Reduktion werden zweckmässig Aether, wie Diäthyläther, Tetrahydrofuran, Dimethoxyäthan und Dioxan, als Lösungsmittel verwendet. Die Reduktion kann bei einer Temperatur zwischen 0 und 100°C, vorzugsweise zwischen 35 und 70°C, durchgeführt werden.

Der Alkohol der Formel V wird in der nächsten Stufe d) in ein Halid oder ein Sulfonatester der Formel VI übergeführt. Verbindungen, worin $X^1$ p-Toluolsulfonyloxy ist, sind bevorzugt.

Zur Herstellung eines Sulfonatesters kann ein Alkohol der Formel V mit einem entsprechend substituierten Sulfonylhalogenid in Gegenwart einer Base umgesetzt werden. Die Herstellung von Verbindungen der Formel VI, worin $X^1$ Jod, Brom oder Chlor ist, kann entweder durch direkte Reaktion eines Alkohols der Formel V mit einem Halogenierungsmittel, wie Phosphortribromid, oder durch Reaktion eines Sulfonatesters der Formel VI mit einem ein Halogenion enthaltenden Verbindung bewerkstelligt werden. So kann ein Tosylat der Formel VI mit einem Alkalimetallbromid oder Jodid, z.B. Kaliumbromid oder -jodid, umgesetzt werden. Alle diese Reaktionen können in an sich bei der Ueberführung von primären Alkoholen zu primären Alkylhalogeniden und Sulfonatestern bekannter Weise durchgeführt werden.

In der Reduktionsstufe e) können die gleichen komplexen Metallhydridreduktionsmittel wie in Stufe c) zweckmässig bei einer Temperatur zwischen Raumtemperatur und 100°C, vorzugsweise zwischen 35 und 70°C, verwendet werden.

Andere in Stufe e) verwendbare Reduktionsmittel, insbesondere für Bromide oder Jodide der Formel VI, sind Alkalimetallcyanborhydride, z.B. Natriumcyanborhydrid; Tri-(niederalkyl)-zinnhydride, z.B. Tri-(n-butyl)-zinnhydrid; Tri-(aryl)-zinnhydride, z.B. Triphenylzinnhydrid, vorzugsweise Tri-(n-butyl)-zinnhydrid. Die Reduktion wird zweckmässigerweise bei einer Temperatur zwischen -20 und +80°C, vorzugsweise zwischen 0 und 40°C, durchgeführt.

In der Stufe e) können die gleichen Aether wie in der Stufe c) als Lösungsmittel verwendet werden.

In der Stufe f) werden zweckmässig anorganische Säuren, wie Salzsäure oder Schwefelsäure; oder organische Sulfonsäuren, wie p-Toluolsulfonsäure, als starke Säuren; Alkohole, wie Methanol und Aethanol, als protische Lösungsmittel; und 2,2-Dimethoxy- oder 2,2-Diäthoxypropan als Ketallösungsmittel verwendet. Die Abspaltung der Schutzgruppen R wird vorzugsweise bei einer Temperatur zwischen -10 und +80°C, vorzugsweise zwischen 0 und 40°C, durchgeführt.

Die Niederalkanoylierung der Stufe g) kann in an sich bekannter Weise durchgeführt werden, z.B. mittels Essigsäureanhydrid und Pyridin, in Gegenwart von Dimethylaminopyridin als Katalysator und bei einer Temperatur zwischen 25 und 100°C.

Die Halogenierungsstufe h) kann mit 1,3-Dibrom-5,5-dimethylhydantoin, N-Chlor- oder N-Bromsuccinimid oder N-Bromacetamid, gelöst in einem gesättigten gegebenenfalls halogenierten aliphatischen Kohlenwasserstoff, wie Hexan oder Tetrachlorkohlenstoff, in Gegenwart eines Neutralisationsmittels wie Natriumbicarbonat oder -carbonat, bei Siedepunkt des Reaktionsgemisches durchgeführt werden. Man erhält ein Gemisch von 7α- und 7β-Halocholesterinen der Formel VIII, das ohne Trennung des 7α- von 7β-Isomeren in der nächsten Stufe verwendet werden kann.

Die Dehydrohalogenierungsstufe i) wird mit heteroaromatischen oder aliphatischen tertiären Aminen, wie Pyridine oder alkylierte Pyridine, z.B. Picoline, Lutidine und Collidine; Triäthylamin, Tripropylamin, 1,5-Diazabicyclo(4.3.0)non-5-en oder 1,4-Diazabicyclo(2.2.2.)octan; vorzugsweise s-Collidin, durchgeführt. In dieser Stufe können auch Trialkylphosphite verwendet werden. Vorzugsweise werden aromatische und aliphatische Lösungsmittel, wie Benzol, Toluol, Xylol oder Decalin, insbesondere Xylol, verwendet. Die Reaktion wird zweckmässig bei Temperaturen zwischen 50°C und Rückflusstemperatur des Reaktionsgemisches, vorzugsweise bei Rückflusstemperatur, durchgeführt. Das erwünschte Steroid-5,7-dien der Formel IX kann durch übliche chemische oder physische Methoden, wie Chromatographie, isoliert werden.

Die Bestrahlungsstufe j) wird unter einer inerten Atmosphäre, mit einer mit einem Kühlungsfinger versehenen Quecksilberlampe, bei einer Temperatur zwischen -40 und +25°C, vorzugsweise bei -5°C, durchgeführt. Als inerte Atmosphäre wird Stickstoff, Helium oder Argon verwendet. Man kann eine Hoch- oder Niederdruckquecksilber, Xenonquecksilber oder Thalliumquecksilberlampe, vorzugsweise eine Hochdruckquecksilberlampe verwenden. Als Lösungsmittel kann man gesättigte aliphatische Kohlenwasserstoffe, wie Pentan, Hexan oder Isooctan; oder Aether, wie Diäthyläther, Dimethoxyäthan, Tetrahydrofuran oder Dioxan, vorzugsweise Gemische von Hexan und Tetrahydrofuran, verwenden.

Nach der Bestrahlung werden die Lösungsmittel abgedampft und der Rückstand wird in das Precholecalciferol der Formel X-A und dem unveränderten Steroid-5,7-dien der Formel IX durch Flüssigchromatographie mit einem festen Absorptionsmittel, wie Porasil, und einem inerten organischen Eluierungsmittel aufgetrennt. Als Eluierungsmittel verwendet man vorzugsweise Gemische von Kohlenwasserstoffe, wie n-Hexan, Isooctan oder Cyclohexan, und Ester, wie Aethylacetat oder -formiat, wobei Gemische von n-Hexan und Aethylacetat bevorzugt sind.

Unveränderges 5,7-Dien der Formel IX wird erneut der Bestrahlung unterworfen und man erhält dabei zusätzliche Mengen des Precholecalciferols der Formel X-A.

Die Verseifungsstufe k) wird mit starken Basen in protischen Lösungsmitteln durchgeführt. Als Base verwendet man zweckmässig Alkali- oder Erdalkalimetallhydroxide oder -alkoxide, wie Methoxide oder Aethoxide, wobei Kaliumhydroxid bevorzugt ist. Als Lösungsmittel verwendet man zweckmässig Alkohole, wie Methanol und Aethanol, und Wasser mit einem mischbaren Cosolvent zur Solubilisierung der organischen Reagentien, z.B. ein Aether, wie Tetrahydrofuran oder Dimethoxyäthan. Methanol ist bevorzugt. Die Verseifung wird vorzugsweise bei einer Temperatur zwischen -20 und +60°C, insbesondere zwischen -5 und +30°C, und unter einer inerten Atmosphäre, wie Stickstoff oder Argon, durchgeführt.

Die Stufe l) wird durch Erhitzen des Precholecalciferolderivates der Formel X-B in einem inerten Lösungsmittel, wie einem Aether, z.B. Dioxan, Tetrahydrofuran oder Dimethoxyäthan; einem aromatischen Kohlenwasserstoff, z.B. Benzol oder Toluol, unter einer inerten Atmosphäre, wie Argon oder Helium, durchgeführt.

Die letzte Stufe m) beträgt die Abspaltung der Ketalschutzgruppe aus dem Cholecalciferolderivat der Formel XI durch Behandlung mit einer Säure in einem protischen Lösungsmittel. Als Säure verwendet man zweckmässig anorganische Säuren, wie Salzsäure oder Schwefelsäure; organische Säuren, wie p-Toluolsulfonsäure oder Trifluoressigsäure; und kationische Austauscherharzen in Wasserstoffform, wobei letztere, z.B. Bio-Rad 50W-X4 bevorzugt sind. Als Lösungsmittel verwendet man zweckmässig Alkanole, wie Methanol, Aethanol oder 2-Propanol; Alkandiole, z.B. Aethylen- oder Propylenglykol; und Wasser mit einem mischbaren Cosolvent, wie einem Aether oder Keton, z.B. Aceton.

Alkanole, insbesondere Methanol, sind bevorzugt. Die Reaktion wird vorzugsweise bei einer Temperatur zwischen -10 und +80°C, insbesondere zwischen 0 und 40°C, durchgeführt.

## Beispiel

A. Herstellung der Ausgangsmaterialien

a) Ein Gemisch von 0,91 g (0,0030 Mol) 1α,3β-Dihydroxy-androst-5-en-17-on, 15 ml Tetrahydrofuran, 1,26 g (0,015 Mol) 3,4-Dihydro-2H-pyran und 0,028 mg p-Toluolsulfonsäuremonohydrat wurde 18 Stunden bei 25°C gerührt. Das Gemisch wurde mit Methylenchlorid verdünnt und dann mit gesättigter Natriumbicarbonatlösung gewaschen. Die organischen Phasen wurden getrocknet, filtriert und zur Trockene eingedampft. Man erhielt 1α,3β-Bis-(tetrahydro-2H-pyran-2-yl-oxy)-androst-3-en-17-on, $[\alpha]_D^{20}$ +34,3° (c 1, $CHCl_3$) in Form eines Oels.

b) Einem Gemisch von 1,00 g (0,0021 Mol) 1α,3β-Bis-(tetrahydro-2H-pyran-2-yl-oxy)androst-3-en-17-on, 1,94 g (0,0087 Mol) Triäthylphosphonoacetat und 14 ml Aethanol wurden 0,68 g (0,010 Mol) Natriumäthoxid in 7 ml Aethanol zugesetzt. Das Gemisch wurde bei 80°C gerührt, dann abgekühlt und unter vermindertem Druck eingeengt. Der Rückstand wurde zwischen Wasser und Aether aufgeteilt. Die organische Phase wurde mit gesättigter Kochsalzlösung gewaschen, dann getrocknet, filtriert und zur Trockene eingedampft. Der Rückstand wurde auf Silicagel chromatographiert und man erhielt [1α,3β,17(20)E]-1,3-Bis(tetrahydro-2H-pyran-2-yl-oxy)-pregna-5,17(20)-dien-21-carbonsäureäthylester, $[\alpha]_D^{20}$ -8° (c 1, $CHCl_3$).

c) Ein Gemisch von 0,32 g (0,00059 Mol) dieses Esters, 0,10 g Platinoxid und 20 ml Aethanol wurde unter 1 Atmosphäre Wasserstoff gerührt und dann filtriert, die Feststoffe wurden mit Aethanol gewaschen und die vereinigten Filtrate wurden zur Trockene eingedampft. Man erhielt 1α,3β-Bis-(tetrahydro-2H-pyran-2-yl-oxy)-pregn-5-en-21-carbonsäureäthylester, $[\alpha]_D^{20}$ -13° (c 1, $CHCl_3$) in Form eines Oels.

B. Herstellung des Produktes

a)1) Einer Lösung von 8,0 ml Diisopropylamin in 14 ml Tetrahydrofuran wurden bei -30°C 29,8 ml (0,0477 Mol) 1,6M Butyllithium in Hexan zugesetzt. Nach Rühren wurden 20,0 g (0,0367 Mol) 1α,3β-Bis-(tetrahydro-2H-pyran-2-yl-oxy)-pregn-5-en-21-carbonsäureäthylester in 180 ml Tetrahydrofuran tropfenweise zugesetzt. Das Gemisch wurde bei -30°C gerührt und auf -70°C abgekühlt. Eine Lösung von 15,64 g (0,0551 Mol) 5-Jod-2S-methylpentan-1,2-diol-1,2-(1-methyläthyliden)-acetal in 22 ml Hexamethylphosphoramid wurde tropfenweise zugesetzt. Das Gemisch wurde bei -70°C gerührt, dann auf 25°C erwärmen gelassen, gerührt und mit 9:1 Hexanäther verdünnt. Die Lösung wurde mit Wasser und gesättigter Kochsalzlösung gewaschen. Die organische Phase wurde getrocknet, filtriert und zur Trockene eingedampft. Der Rückstand wurde durch Chromatographie auf Silicagel gereinigt. Man erhielt [1α,3β,25S]-1,3-Bis-(tetrahydro-2H-pyran-2-yl-oxy)-cholest-5-en-21-carbonsäure-25,26-diol-25,26-(1-methyläthyliden)-acetal-äthylester, $[\alpha]_D^{20}$ +6,7° (c 1, $CHCl_3$).

a)2) In zu a)1) ähnlicher Weise wurden 8,50 g (0,0156 Mol) 1α,3β-Bis-(tetrahydro-2H-pyran-2-yl-oxy)-pregn-5-en-21-carbonsäureäthylester mit 6,65 g (0,0234 Mol) 5-Jod-2R-methylpentan-1,2-diol-1,2-(1-methyläthylidenacetal umgesetzt. Man erhielt [1α,3β,25R]-1,3-Bis-(tetrahydro-2H-pyran-2-yl-oxy)-cholest-5-en-21-carbonsäure-25,26-diol-25,26-(1-methyläthyliden)-acetal-äthylester, $[\alpha]_D^{20}$ +8,3° (c 1, $CHCl_3$).

b)1) Einem Gemisch von 2,05 g (0,0090 Mol) Lithiumaluminiumhydrid und 100 ml Tetrahydrofuran wurden bei 0°C 25,2 g [1α,3β,25S]-1,3-Bis-(tetrahydro-2H-pyran-2-yl-oxy)-cholest-5-en-21-carbonsäure-25,26-diol-25,26-(1-methyläthyliden)-acetal-äthylester in 250 ml Tetrahydrofuran zugesetzt. Das Gemisch wurde auf 60°C erhitzt, auf 0°C abgekühlt und mit 900 ml Aether verdünnt. Dem Gemisch wurden

tropfenweise 4,10 ml Wasser und 3,30 ml 10%-iger Natriumhydroxidlösung zugesetzt. Das Gemisch wurde bei 25°C gerührt und filtriert. Die Feststoffe wurden mit Aether zerrieben und filtriert. Nach Abdampfen des Lösungsmittels erhielt man [1α,3β,25S]-1,3-Bis-(tetrahydro-2H-pyran-2-yl-oxy)-cholest-5-en-21,25,26-triol-25,26-(1-methyläthyliden)-acetal, $[\alpha]_D^{20}$ -4,6° (c 1, $CHCl_3$).

b)2) In zu b)1) ähnlicher Weise wurden 10,5 g (0,0150 Mol) [1α,3β,25R]-1,3-Bis-(tetrahydro-2H-pyran-2-yl-oxy)-cholest-5-en-21-carbonsäure-25,26-diol-25,26-(1-methyläthyliden)-acetal-äthylester mit 0,853 g (0,0225 Mol) Lithiumaluminiumhydrid umgesetzt. Man erhielt [1α,3β,25R]-1,3-Bis-(tetrahydro-2H-pyran-2-yl-oxy)-cholest-5-en-21,25,26-triol-25,26-(1-methyläthyliden)-acetal, $[\alpha]_D^{20}$ -0,6° (c 1, $CHCl_3$).

c)1) Ein Gemisch von 24,20 g (0,0358 Mol) [1α,3β,25S]-1,3-Bis-(tetrahydro-2H-pyran-2-yl-oxy)-cholest-5-en-21,25,26-triol-25,26-(1-methyläthyliden)-acetal, 150 ml Pyridin und 13,52 g (0,0708 Mol) p-Toluolsulfonylchlorid wurde bei 0°C gerührt, mit Eisstücken abgekühlt, dann in Wasser geschüttet und mit Methylenchlorid extrahiert. Die organische Phase wurde mit 10%-iger Schwefelsäure und gesättigter Natriumbicarbonatlösung gewaschen. Die organische Schicht wurde getrocknet, filtriert und zur Trockene eingedampft. Man erhielt [1α,3β,25S]-1,3-Bis-(tetrahydro-2H-pyran-2-yl-oxy)-cholest-5-en-21,25,26-triol-25,26-(1-methyläthyliden)-acetal-21-(4-methylbenzolsulfonat), $[\alpha]_D^{22}$ -3,7° (c 1, $CHCl_3$) in Form eines Oels.

c)2) In zu c)1) analoger Weise wurden 10,24 g (0,0155 Mol) [1α,3β,25R]-1,3-Bis-(tetrahydro-2H-pyran-2-yl-oxy)-cholest-5-en-21,25,26-triol-25,26-(1-methyläthyliden)-acetal mit 5,94 g (0,0311 Mol) p-Toluolsulfonylchlorid umgesetzt. Man erhielt [1α,3β,25R]-1,3-Bis-(tetrahydro-2H-pyran-2-yl-oxy)-cholest-5-en-21,25,26-triol-25,26-(1-methyläthyliden)-acetal-21-(4-methylbenzolsulfonat), $[\alpha]_D^{23}$ +2,1°

(c 1, $CHCl_3$) in Form eines Oels.

d)1) Ein Gemisch von 0,081 g (0,0001 Mol)[1α,3β,25S]-1,3-Bis-(tetrahydro-2H-pyran-2-pyran-2-yl-oxy)-cholest-5-en-21,25,26-triol-25,26-(1-methyläthyliden)-acetal-21-(4-methylbenzolsulfonat) und 0,150 g (0,0010 Mol) Natriumjodid in 2 ml Aceton wurde bei 50°C erhitzt, dann abgekühlt und in Wasser gegossen. Das Produkt wurde mit Methylenchlorid isoliert, die organischen Schichten wurden mit einer Natriumsulfitlösung und gesättigter Natriumbicarbonatlösung gewaschen, dann getrocknet, filtriert und zur Trockene eingedampft. Man erhielt [1α,3β,25S]-1,3-Bis-(tetrahydro-2H-pyran-2-yl-oxy)-21-jodcholest-5-en-25,26-diol-25,26-(1-methyläthyliden)-acetal.

d)2) In zu d)1) analoger Weise wurden 0,081 g (0,0001 Mol) [1α,3β,25R]-1,3-Bis-(tetrahydro-2H-pyran-2-yl-oxy)-cholest-5-en-21,25,26-triol-25,26-(1-methyläthyliden)-acetal-21-(4-methylbenzolsulfonat) mit 0,165 g (0,0011 Mol) Natriumjodid umgesetzt. Man erhielt [1α,3β,25R]-1,3-Bis-(tetrahydro-2H-pyran-2-yl-oxy)-21-jodcholest-5-en-25,26-diol-25,26-(1-methyläthyliden)-acetal.

e)1) Ein Gemisch von 4,43 g (0,1166 Mol) Lithiumaluminiumhydrid, 410 ml Tetrahydrofuran und 31,60 g (0,0357 Mol) [1α,3β,25S]-1,3-Bis-(tetrahydro-2H-pyran-2-yl-oxy)-cholest-5-en-21,25,26-triol-25,26-(1-methyläthyliden)-acetal-21-(4-methylbenzolsulfonat) wurde unter Rückfluss (60°C) erhitzt, dann auf 0°C abgekühlt, mit 900 ml Aether verdünnt und mit 8,86 ml Wasser und 7,09 ml 10%-iger Natriumhydroxidlösung versetzt. Das Gemisch wurde gerührt und filtriert. Die Feststoffe wurden mit Aether zerrieben und filtriert. Die vereinigten Filtrate wurden zur Trockene eingedampft und auf Silicagel chromatographiert. Man erhielt [1α,3β,25S]-1,3-Bis-(tetrahydro-2H-pyran-2-yl-oxy)-cholest-5-en-25,26-diol-25,26-(1-methyläthyliden)-acetal, $[\alpha]_D^{22}$ -6,6° (c 1, $CHCl_3$).

e)2) In einer Verfahrensvariante wurde ein Gemisch von 0,078 g (0,0001 Mol) [1α,3β,25S]-1,3-Bis-(tetrahydro-2H-pyran-2-yl-oxy)-21-jodcholest-5-en-25,26-diol-25,26-(1-methyläthyliden)-acetal, 0,045 g (0,00015 Mol) tri-n-Butylzinnhydrid und 3 ml Tetrahydrofuran unter einer Argonatmosphäre bei 25°C gerührt und dann zur Trockene eingedampft. Der Rückstand wurde durch Chromatographie auf Silicagel gereinigt. Man erhielt [1α,3β,25S]-1,3-Bis-(tetrahydro-2H-pyran-2-yl-oxy)-cholest-5-en-25,26-diol-25,26-(1-Methyläthyliden)-acetal, $[\alpha]_D^{23}$ -6,5° (c 1, $CHCl_3$).

e)3) In einer zu e)1) analoger Weise wurden 1,93 g (0,0509 Mol) Lithiumaluminiumhydrid mit 13,46 g (0,0166 Mol) [1α,3β,25R]-1,3-Bis-(tetrahydro-2H-pyran-2-yl-oxy)-cholest-5-en-21,25,26-triol-25,26-(1-methyläthyliden)-acetal-21-(4-methylbenzolsulfonat) umgesetzt. Man erhielt [1α,3β,25R]-1,3-Bis-(tetrahydro-2H-pyran-2-yl-oxy)-cholest-5-en-25,26-diol-25,26-(1-methyläthyliden)-acetal, $[\alpha]_D^{25}$ -2,5° (c 1, $CHCl_3$).

e)4) In zu e)2) ähnlicher Weise wurden 0,076 g (0,0001 Mol) [1α,3β,25R]-1,3-Bis-(tetrahydro-2H-pyran-2-yl-oxy)-21-jodcholest-5-en-25,26-diol-25,26-(1-methyläthyliden)-acetal mit 0,044 g (0,00015 Mol) tri-n-Butylzinnhydrid umgesetzt. Man erhielt [1α,3β,25R]-1,3-Bis-(tetrahydro-2H-pyran-2-yl-oxy)-cholest-5-en-25,26-diol-25,26-(1-methyläthyliden)-acetal, $[\alpha]_D^{25}$ -2,5° (c 1, $CHCl_3$).

f)1) Ein Gemisch von 24,71 g (0,0355 Mol) [1α,3β,25S]-1,3-Bis-(tetrahydro-2H-pyran-2-yl-oxy)-cholest-5-en-25,26-diol-25,26-(1-methyläthyliden)-acetal, 420 ml Methanol, 420 ml 2,2-Dimethoxypropan und 4,20 g p-Toluolsulfonsäuremonohydrat wurde bei 25°C gerührt, dann mit gesättigter Natriumbicarbonatlösung unter Rühren versetzt und zur Trockene eingedampft. Der Rückstand wurde mit Aethylacetat zerrieben, filtriert und zur Trockene eingedampft. Der rohe Feststoff wurde durch Chromatographie auf Silicagel gereinigt. Man erhielt [1α,3β,25S]-Cholest-5-en-1,3,25,26-tetrol-25,26-(1-methyläthyliden)-acetal, Smp. 175-177°, $[\alpha]_D^{21}$ -39,9° (c 1, $CHCl_3$).

f)2) In einer zu f)1) ähnlicher Weise wurden 10,65 g (0,0165 Mol) [1α,3β,25R]-1,3-Bis-(tetrahydro-2H-pyran-2-yl-oxy)-cholest-5-en-25,26,diol-25,26-(1-methyläthyliden)-acetal mit 1,80 g p-Toluolsulfonsäure umgesetzt. Man erhielt [1α,3β,25R]-Cholest-5-en-1,3,25,26-tetrol-25,26-(1-methyläthyliden)-acetal, Smp. 151-153°C, $[\alpha]_D^{25}$ -27,9° (c 1, $CHCl_3$).

g)1) Ein Gemisch von 10,93 g (0,0230 Mol) [1α,3β,25S]-Cholest-5-en-1,3,25,26-tetrol-25,26-(1-methyläthyliden)-acetal, 82 ml Pyridin, 21 ml Essigsäureanhydrid und 1,09 g 4-Dimethylaminopyridin wurde bei 0°C und dann bei 25°C gerührt, mit 20 ml Methanol bei 0°C verdünnt und zur Trockene eingedampft. Der Rückstand wurde in Methylenchlorid gelöst. Die Lösung wurde mit 10%-iger Schwefelsäure und gesättigter Natriumbicarbonatlösung gewaschen. Die organische Phase wurde getrocknet, filtriert und zur Trockene eingedampft. Man erhielt [1α,3β,25S]-Cholest-5-en-1,3,25,26-tetrol-25,26-(1-methyläthyliden)-acetal-1,3-diacetat, $[\alpha]_D^{21}$-19,0° (c 1, $CHCl_3$).

g)2) In zu g)1) ähnlicher Weise wurden 4,58 g (0,0097 Mol) [1α,3β,25R]-Cholest-5-en-1,3,25,26-tetrol-25,26-(1-methyläthyliden)-acetal mit 8,8 ml Essigsäureanhydrid umgesetzt. Man erhielt [1α,3β,25R]-Cholest-5-en-1,3,25,26-tetrol-25,26-(1-methyläthyliden)-acetal-1,3-diacetat, $[\alpha]_D^{20}$ -14,4° (c 1, $CHCl_3$).

h)1) Ein Gemisch von 12,79 g (0,0228 Mol) [1α,3β,25S]-Cholest-5-en-1,3,25,26-tetrol-25,26-(1-methyläthyliden)-acetal-1,3-diacetat, 10,31 g Natriumbicarbonat, 4,38 g (0,0150 Mol) 1,3-Dibrom-5,5-dimethylhydantoin und 500 ml Hexan wurde bei Rückflusstemperatur (80°C) erhitzt, dann abgekühlt und filtriert. Die Feststoffe wurden mit Hexan zerrieben und filtriert. Die Filtrate wurden zur Trockene

eingedampft. Man erhielt [1α,3β,25S]-7-Bromcholest-5-en-1,3,25,26-tetrol-25,26-(1-methyläthyliden)-acetal-1,3-diacetat.

h)2) In zu h)1) ähnlicher Weise wurden 5,35 g (0,0096 Mol) [1α,3β,25R]-Cholest-5-en-1,3,25,26-tetrol-25,26-(1-methyläthyliden)-acetal-1,3-diacetat mit 1,84 g (0,00630 Mol) 1,3-Dibrom-5,5-dimethylhydantoin umgesetzt. Man erhielt [1α,3β,25R]-7-Bromcholest-5-en-1,3,25,26-tetrol-25,26-(1-methyläthyliden)-acetal-1,3-diacetat.

i)1) Ein Gemisch von 13,2 g (0,0228 Mol) rohes [1α,3β,25S]-7-Bromcholest-5-en-1,3,25,26-tetrol-25,26-(1-methyläthyliden)-acetal-1,3-diacetat, 9,37 ml s-Collidin und 120 ml Xylol wurde bei Rückflusstemperatur (140°C) erhitzt, dann abgekühlt und mit 350 ml Hexan verdünnt. Die Lösung wurde mit 10%-iger Schwefelsäure und gesättigter Natriumbicarbonatlösung gewaschen. Die organische Phase wurde getrocknet, filtriert und zur Trockene eingedampft. Der Rückstand wurde durch Chromatohraphie auf Silicagel gereinigt. Man erhielt [1α,3β,25S]-Cholesta-5,7-dien-1,3,25,26-tetrol-25,26-(1-methyläthyliden)-acetal-1,3-diacetat, Smp. 105-107°C, $[\alpha]_D^{24}$ -27,9° (c 1, $CHCl_3$).

i)2) In zu i)1) ähnlicher Weise wurden 6,00 g (0,0096 Mol) rohes [1α,3β,25R]-7-Bromcholest-5-en-1,3,25,26-tetrol-25,26-(1-methyläthyliden)-acetal-1,3-diacetat mit 3,9 ml s-Collidin umgesetzt. Man erhielt [1α,3β,25R]-Cholesta-5,7-dien-1,3,25,26-tetrol-25,26-(1-methyläthyliden)-acetal-1,3-diacetat, $[\alpha]_D^{20}$ -24,9° (c 1, $CHCl_3$).

j)1) Ein Gemisch von 1,74 g (0,0031 Mol) [1α,3β,25S]-Cholesta-5,7-dien-1,3,25,26-tetrol-25,26-(1-methyläthyliden)-acetal-1,3-diacetat in 600 ml Hexan und 200 ml Tetrahydrofuran wurde unter Argon bei -5°C mit einer mit einem Kühlfinger aus Glas abgekühlten Hochdruckquecksilberlampe bestrahlt. Die Lösung wurde zur Trockene eingedampft und der Rückstand mit Silicagel und einem Gemisch von 7:1

n-Hexan-äthylacetat chromatographiert. Man erhielt [1α,3β,6Z,25S]-9,10-Secocholesta-5(10),6,8-trien-1,3,25,26-tetrol-25,26-(1-methyläthyliden)-acetal-1,3-diacetat, $[\alpha]_D^{23}$ -52,2° (c 0,5, $CHCl_3$).

j)2) In einer zu j)1) ähnlicher Weise wurden 1,90 g (0,0034 Mol) [1α,3β,25R]-Cholesta-5,7-dien-1,3,25,26-tetrol-25,26-(1-methyläthyliden)-acetal-1,3-diacetat in [1α,3β,6Z,25R]-9,10-Secocholesta-5(10),6,8-trien-1,3,25,26-tetrol-25,26-(1-methyläthyliden)-acetal-1,3-diacetat, $[\alpha]_D^{20}$ -52,0° (c 1, $CHCl_3$) übergeführt.

k)1) Eine Lösung von 0,443 g (0,0080 Mol) [1α,3β,6Z,25S]-9,10-Secocholesta-5(10),6,8-trien-1,3,25,26-tetrol-25,26-(1-methyläthyliden)-acetal-1,3-diacetat und 15 ml 1,5%-iger Kaliumhydroxidlösung in Methanol wurde bei 0°C gerührt, dann mit Eisessig in Methanol auf pH 7,5 neutralisiert und zur Trockene eingedampft. Der Rückstand wurde zwischen Aethylacetat und Wasser aufgeteilt. Die organische Phase wurde mit gesättigter Salzlösung gewaschen und getrocknet. Das Gemisch wurde filtriert und zur Trockene eingedampft. Man erhielt [1α,3β,6Z,25S]-9,10-Secocholesta-5(10),6,8-trien-1,3,25,26-tetrol-25,26-(1-mehyläthyliden)-acetal.

k)2) In einer zu k)1) ähnlicher Weise wurde 0,515 g (0,00093 Mol) [1α,3β,6Z,25R]-9,10-Secocholesta-5(10),6,8-trien-1,3,25,26-tetrol-25,26-(1-methyläthyliden)-acetal-1,3-diacetat mit 20 ml 1,5%-igem Kaliumhydroxidlösung in Methanol umgesetzt. Man erhielt [1α,3β,6Z,25R]-9,10-Secocholesta-5(10),6,8-trien-1,3,25,26-tetrol-25,26-(1-methyläthyliden)-acetal.

l)1) Ein Gemisch von 0,174 g (0,0003 Mol) [1α,3β,6Z,25S]-9,10-Secocholesta-5(10),6,8-trien-1,3,25,26-tetrol-25,26-(1-methyläthyliden)-acetal und 5 ml Dioxan wurde bei Rückflusstemperatur (100°C) erhitzt, abgekühlt und dann zur Trockene eingedampft. Der Rückstand wurde durch Chromatographie mit Silicagel und 3:1 Aethylacetat-Hexan

gereinigt. Man erhielt [1α,3β,5Z,7E,25S]-9,10-Secocholesta-5,7,10(19)-trien-1,3,25,26-tetrol-25,26-(1-methyläthyliden)-acetal, $[\alpha]_D^{23}$+26,9° (c 0,5, $CHCl_3$).

l)2) In zu l)1) ähnlicher Weise wurden 0,440 g (0,0009 Mol) [1α,3β,6Z,25R]-9,10-Secocholesta-5(10),6,8-trien-1,3,25,26-tetrol-25,26-(1-methyläthyliden)-acetal in 15 ml Dioxan erhitzt. Man erhielt [1α,3β,5Z,7E,25R]-9,10-Secocholesta-5,7,10(19)-trien-1,3,25,26-tetrol-25,26-(1-methyläthyliden)-acetal, $[\alpha]_D^{20}$ +28,4° (c 0,5, $CHCl_3$).

m)1) Ein Gemisch von 0,222 g (0,0005 Mol) [1α,3β,5Z,7E,-25S]-9,10-Secocholesta-5,7,10(19)-trien-1,3,25,26-tetrol-25,26-(1-methyläthyliden)-acetal, 0,500 g eines Kationenaustauscherharzes in Wasserstoffform (Bio-Rad AG 50W-X4) und 37 ml Methanol wurde unter Argon bei 20°C gerührt und dann filtriert. Das Filtrat wurde zur Trockene eingedampft. Der Rückstand wurde durch Chromatographie mit Silicagel und Aethylacetat gereinigt. Man erhielt [1α,3β,5Z,7E,25S]-9,10-Secocholesta-5,7,10(19)-trien-1,3,25,26-tetrol auch als 1α,25S,26-Trihydroxycholecalciferol bekannt, Smp. 162-164°C, $[\alpha]_D^{23}$ +58,8° (c 0,5, $CH_3OH$).

m)2) In zu m)1) ähnlicher Weise wurden 0,310 g (0,0007 Mol) [1α,3β,5Z,7E,25R]-9,10-Secocholesta-5,7,10(19)-trien-1,3,25,26-tetrol-25,26-(1-methyläthyliden)-acetal mit 0,718 g eines Kationenaustauscherharzes in Wasserstoffform (Bio-Rad AG 50W-X4) umgesetzt. Man erhielt [1α,3β,5Z,7E,25R]-9,10-Secocholesta-5,7,10(19)-trien-1,3,25,26-tetrol auch als 1α,25R,26-Trihydroxycholecalciferol bekannt, Smp. 146-148°C, $[\alpha]_D^{21}$ +63,8° (c 0,5, $CH_3OH$).

Patentansprüche

1. Verfahren zur Herstellung des 1α,25,26-Trihydroxy-cholecalciferols in Form des C-25 R- or S-Epimeren oder in Form von Gemischen davon, dadurch gekennzeichnet, dass man

a) eine Verbindung der Formel

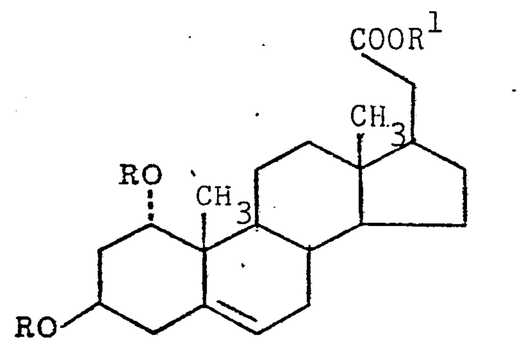

I

worin $R^1$ Niederalkyl, Phenyl oder substituiertes Phenyl und R eine Gruppe der Formel

$$-C(R^2,R^3,OR^4)$$

$R^2$ Wasserstoff oder Niederalkyl und $R^3$ und $R^4$ Niederalkyl oder zusammen $C_{3-6}$-Alkylen sind, mit einem Lithium-, Natrium-, Kalium-, Magnesium- oder Zinkorganometallischen Reagenzien umsetzt,

b) das Produkt der Stufe a) der Formel

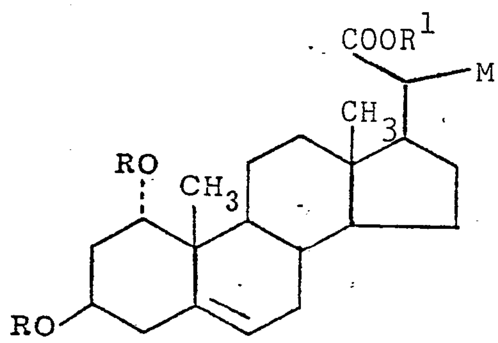

II

worin R und $R^1$ die obige Bedeutung haben und M Lithium, Natrium, Kalium, Magnesium/2 oder Zink/2 sind,

mit einer Verbindung der Formel

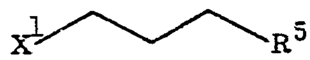

III

worin $X^1$ Jod, Brom, Chlor, Niederalkylsulfonyloxy, Phenylsulfonyloxy oder substituiertes Phenylsulfonyloxy und $R^5$ eine Gruppe der Formel

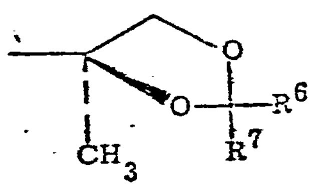

oder

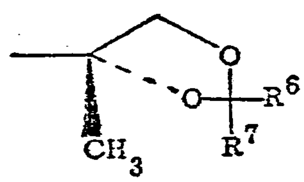

$R^6$ und $R^7$ Niederalkyl oder zusammen Niederalkylen sind,

c) den Ester von Stufe b) der Formel

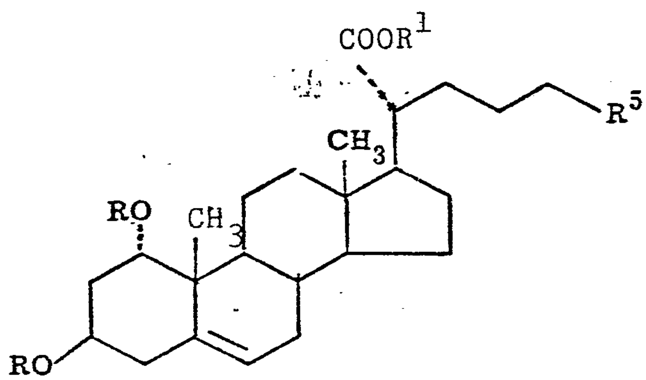

IV

worin R, $R^1$ und $R^5$ die obige Bedeutung haben, reduziert,

d) in dem Produkt der Stufe c) und der Formel

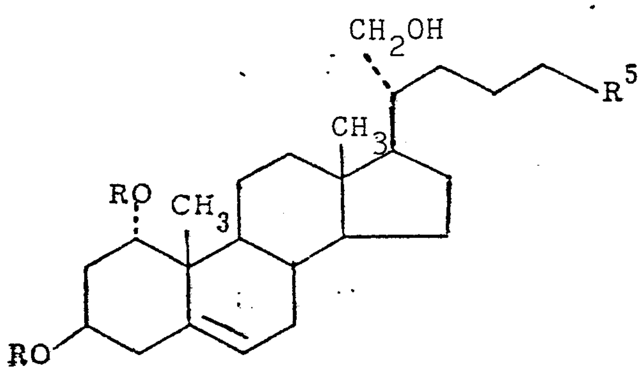

V

worin R und $R^5$ die obige Bedeutung haben, die Hydroxygruppe durch eine Gruppe $X^1$, worin $X^1$ obige Bedeutung hat, ersetzt,

e) das Produkt von Stufe d) und der Formel

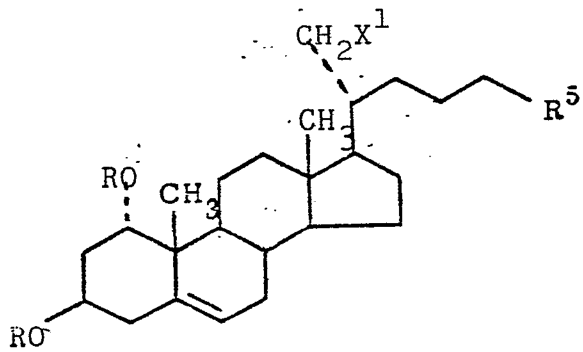

VI

worin R, $R^5$ und $X^1$ die obige Bedeutung haben, mit einem Reduktionsmittel umsetzt,

f) das Produkt der Stufe e) und der Formel

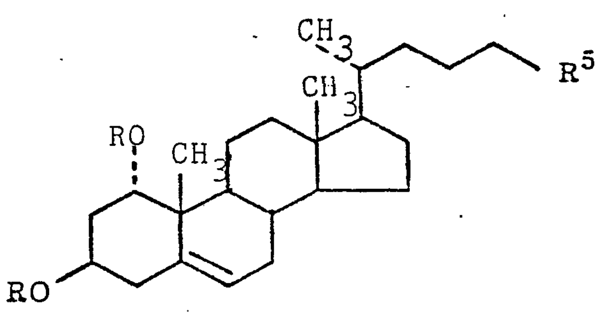

VII-A

worin R und $R^5$ die obige Bedeutung haben, mit einer starken Säure in einem Gemisch eines protischen Lösungsmittels und eines Ketallösungsmittels umsetzt,

g) das Produkt der Stufe f) und der Formel

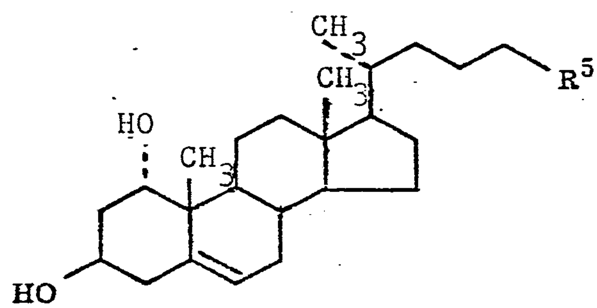

VII-B

worin $R^5$ obige Bedeutung hat, mit einem Niederalkanoylierungsmittel umsetzt,

h) das Produkt der Stufe g) und der Formel

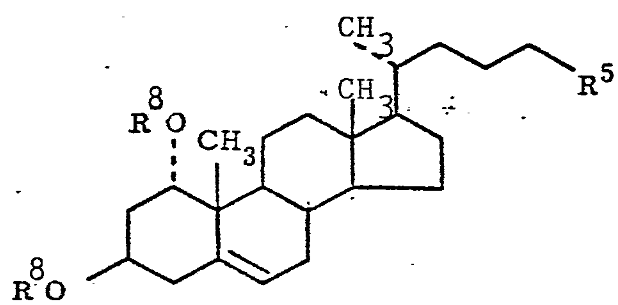

VII-C

worin $R^5$ die obige Bedeutung hat und $R^8$ Niederalkanoyl ist, in 7-Stellung allylisch halogeniert,

i) das Produkt der Stufe h) und der Formel

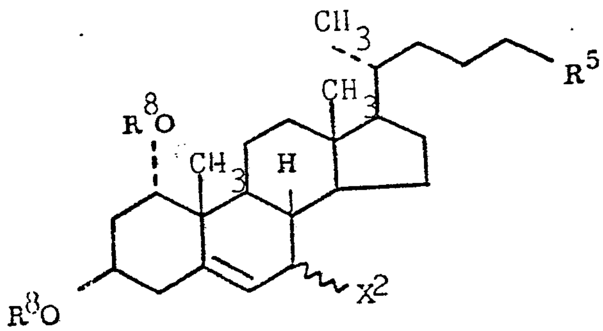

VIII

worin $R^5$ und $R^8$ die obige Bedeutung haben und $X^2$ Brom, Chlor oder Jod ist,
dehydrohalogeniert,

j) das Produkt der Stufe i) und der Formel

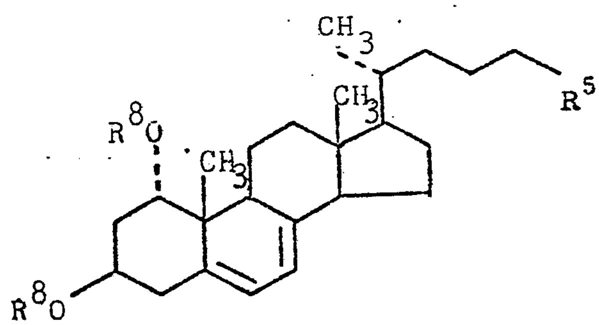

IX

worin $R^5$ und $R^8$ obige Bedeutung haben,
bestrahlt,

k) das Produkt der Stufe j) und der Formel

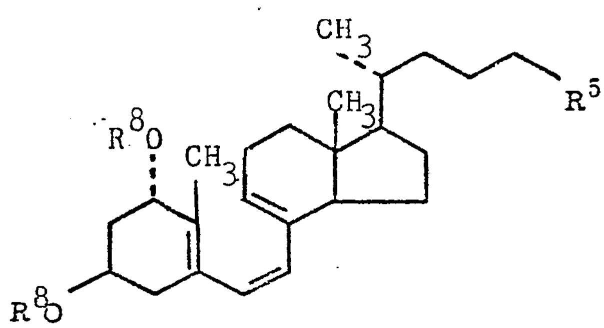

X-A

worin $R^5$ und $R^8$ obige Bedeutung haben, verseift,

l) das Produkt der Stufe k) und der Formel

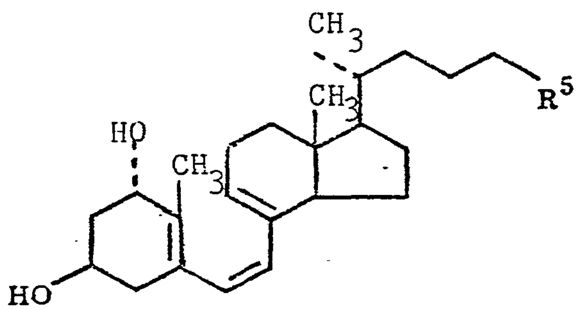

X-B

worin $R^5$ obige Bedeutung hat, thermisch isomerisiert und

m) das Produkt der Stufe l) und der Formel

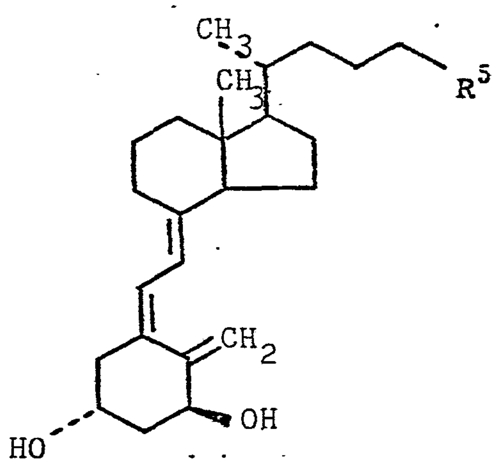

XI

worin $R^5$ obige Bedeutung hat, mit einer Säure in einem protischen Lösungsmittel behandelt.

2. Eine Verbindung in Form des C-25 R- oder S-Epimeren oder in Form von Gemischen davon und der Formel

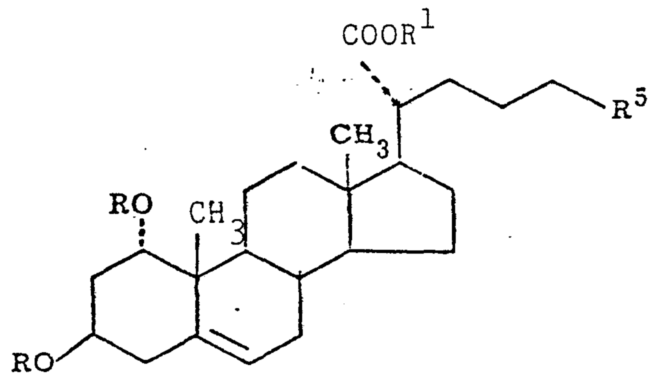

IV

worin $R^1$ Niederalkyl, Phenyl oder substituiertes Phenyl, R eine Gruppe der Formel

$$-C(R^2, R^3, OR^4)$$

$R^2$ Wasserstoff oder Niederalkyl, $R^3$ und $R^4$ Niederalkyl oder zusammen $C_{3-6}$-Alkylen, $R^5$ eine Gruppe der Formel

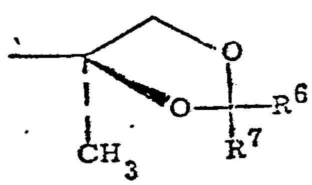

oder

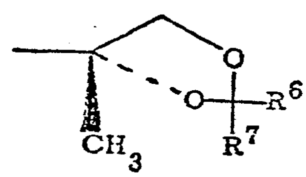

und $R^6$ und $R^7$ Niederalkyl oder zusammen Niederalkylen sind.

3. [1α,3β,25S]-1,3-Bis-(tetrahydro-2H-pyran-yl-oxy)-cholest-5-en-21-carbonsäure-25,26-diol-25,26-(1-methyläthyliden)-acetal-äthylester.

4. [1α,3β,25R]-1,3-Bis-(tetrahydro-2H-pyran-yl-oxy)-cholest-5-en-21-carbonsäure-25,26-diol-25,26-(1-methyläthyliden)-acetal-äthylester.

5. Eine Verbindung in Form des C-25 R- oder S-Epimeren oder in Form eines Gemisches davon und der Formel

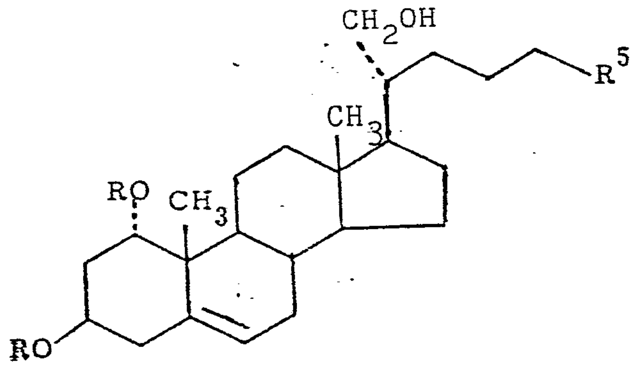

V

worin R eine Gruppe der Formel

$$-C(R^2,R^3,OR^4)$$

$R^2$ Wasserstoff oder Niederalkyl, $R^3$ und $R^4$ Niederalkyl oder zusammen $C_{3-6}$-Alkylen, $R^5$ eine Gruppe der Formel

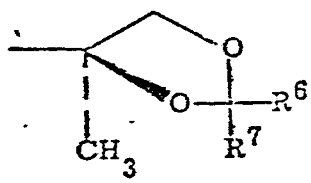

oder

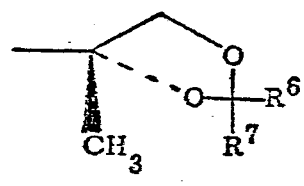

und $R^6$ und $R^7$ Niederalkyl oder zusammen Niederalkylen sind.

6. [1α,3β,25S]-1,3-Bis-(tetrahydro-2H-pyran-2-yl-oxy)-cholest-5-en-21,25,26-triol-25,26-(1-methyläthyliden)-acetal.

7. [1α,3β,25R]-1,3-Bis-(tetrahydro-2H-pyran-2-yl-oxy)-cholest-5-en-21,25,26-triol-25,26-(1-methyläthyliden)-acetal.

8. Eine Verbindung in Form des C-25 R- oder S-Epimeren oder in Form eines Gemisches davon und der Formel

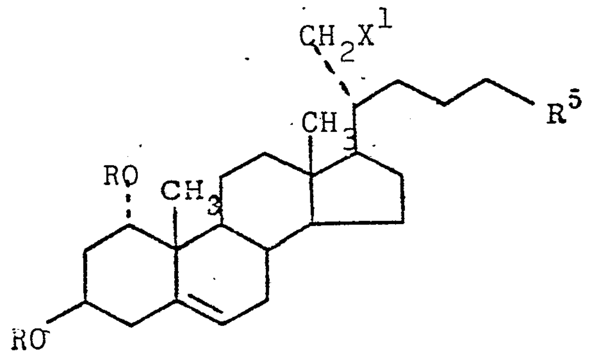

VI

worin $X^1$ Jod, Brom, Chlor, Niederalkylsulfonyloxy, Phenylsulfonyloxy oder substituiertes Phenylsulfonyloxy; R eine Gruppe der Formel

$$-C(R^2, R^3, OR^4)$$

$R^2$ Wasserstoff oder Niederalkyl, $R^3$ und $R^4$ Niederalkyl oder zusammen $C_{3-6}$-Alkylen, $R^5$ eine Gruppe der Formel

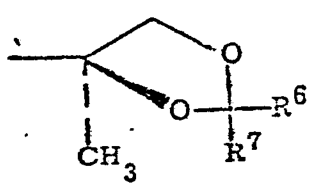 oder 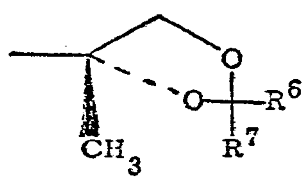

$R^6$ und $R^7$ Niederalkyl oder zusammen Niederalkylen sind.

9. [1α,3β,25S]-1,3-Bis-(tetrahydro-2H-pyran-2-yl-oxy)-cholest-5-en-21,25,26-triol-25,26-(1-methyläthyliden)-acetal-21-(4-methylbenzolsulfonat).

10. [1α,3β,25R]-1,3-Bis-(tetrahydro-2H-pyran-2-yl-oxy)-cholest-5-en-21,25,26-triol-25,26-(1-methyläthyliden)-acetal-21-(4-methylbenzolsulfonat).

11. [1α,3β,25S]-1,3-Bis-(tetrahydro-2H-pyran-2-yl-oxy)-21-jodcholest-5-en-25,26-diol-25,26-(1-methyläthyliden)-acetal.

12. [1α,3β,25R]-1,3-Bis-(tetrahydro-2H-pyran-2-yl-oxy)-21-jodcholest-5-en-25,26-diol-25,26-(1-methyläthyliden)-acetal.

13. Eine Verbindung in Form des C-25 R- oder S-Epimeren oder in Form eines Gemisches davon und der Formel

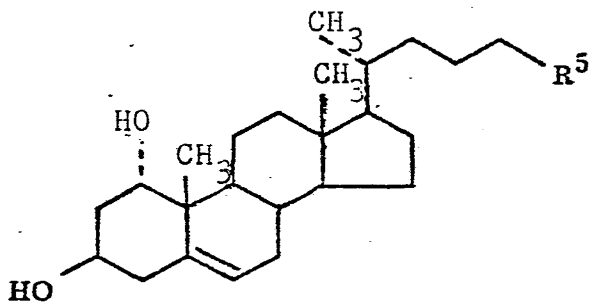

VII-B

worin $R^5$ eine Gruppe der Formel

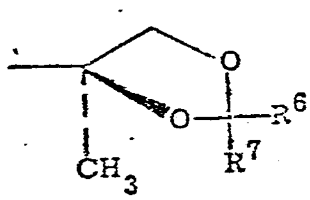

oder

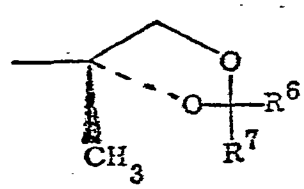

und $R^6$ und $R^7$ Niederalkyl oder zusammen Niederalkylen sind.

14. [1α,3β,25S]-Cholest-5-en-1,3,25,26-tetrol-25,26-(1-methyläthyliden)-acetal.

15. [1α,3β,25R]-Cholest-5-en-1,3,25,26-tetrol-25,26-(1-methyläthyliden)-acetal.

16. Eine Verbindung in Form des C-25 R- oder S-Epimeren oder in Form eines Gemisches davon und der Formel

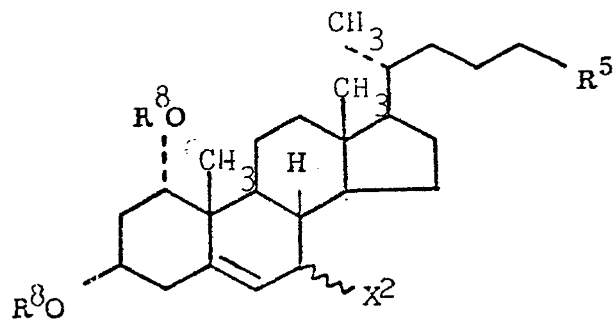

VIII

worin $X^2$ Brom, Chlor oder Jod, $R^8$ Niederalkanoyl, $R^5$ eine Gruppe der Formel

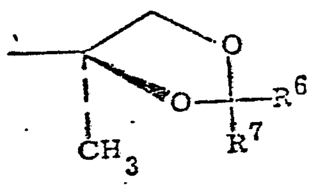

oder

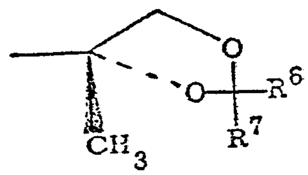

und $R^6$ und $R^7$ Niederalkyl oder zusammen Niederalkylen sind.

17. [1α,3β,7E,25S]-7-Brom-cholest-5-en-1,3,25,26-tetrol-25,26-(1-methyläthyliden)-acetal-1,3-diacetat.

18. [1α,3β,7E,25R]-7-Brom-cholest-5-en-1,3,25,26-tetrol-25,26-(1-methyläthyliden)-acetal-1,3-diacetat.

19. Eine Verbindung in Form des C-25 R- oder S-Epimeren oder in Form eines Gemisches davon und der Formel

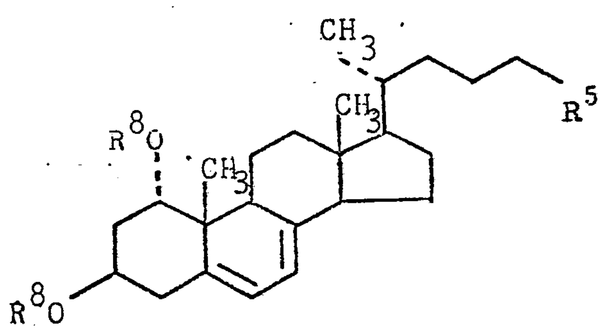

IX

worin $R^8$ Niederalkanoyl und $R^5$ eine der Gruppe

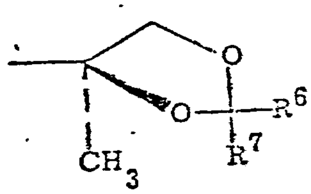 oder 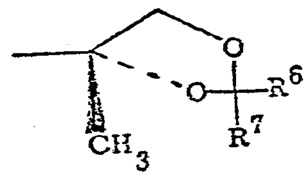

und $R^6$ und $R^7$ Niederalkyl oder zusammen Niederalkylen sind.

20. [1α,3β,25S]-Cholesta-5,7-dien-1,3,25,26-tetrol-25,26-(1-methyläthyliden)-acetal-1,3-diacetat.

21. [1α,3β,25R]-Cholesta-5,7-dien-1,3,25,26-tetrol-25,26-(1-methyläthyliden)-acetal-1,3-diacetat.

22. Eine Verbindung in Form des C-25 R- oder S-Epimeren oder in Form eines Gemisches davon und der Formel

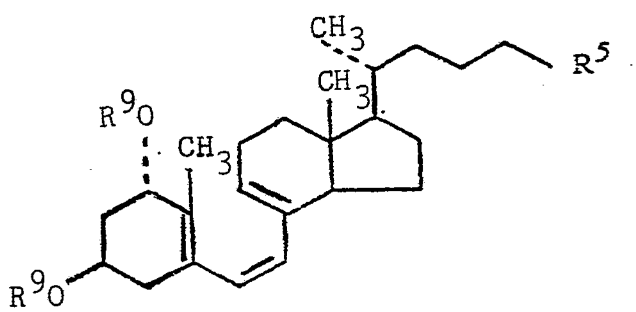

X

worin $R^9$ Wasserstoff oder Niederalkanoyl, $R^5$ eine Gruppe der Formel

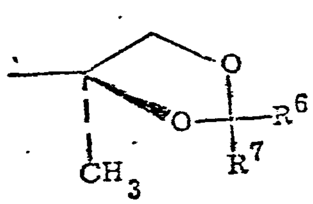 oder 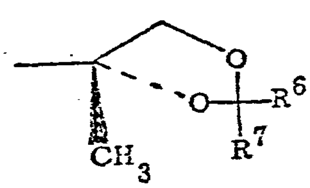

und $R^6$ und $R^7$ Niederalkyl oder zusammen Niederalkylen sind.

23. [1α,3β,6Z,25S]-9,10-Secocholesta-5(10),6,8-trien-1,3,25,26-tetrol-25,26-(1-methyläthyliden)-acetal.

24. [1α,3β,6Z,25R]-9,10-Secocholesta-5(10),6,8,trien-1,3,25,26-tetrol-25,26-(1-methyläthyliden)-acetal.

25. [1α,3β,6Z,25S]-9,10-Secocholesta-5(10),6,8,-trien-1,3,25,26-tetrol-25,26-(1-methyläthyliden)-acetal-1,3-diacetat.

26. [1α,3β,6Z,25R]-9,10-Secocholesta-5(10)-6,8-trien-1,3,25,26-tetrol-25,26-(1-methyläthyliden)-acetal-1,3-diacetat.

27. Eine Verbindung in Form des C-25 R- oder S-Epimeren oder in Form eines Gemisches davon und der Formel

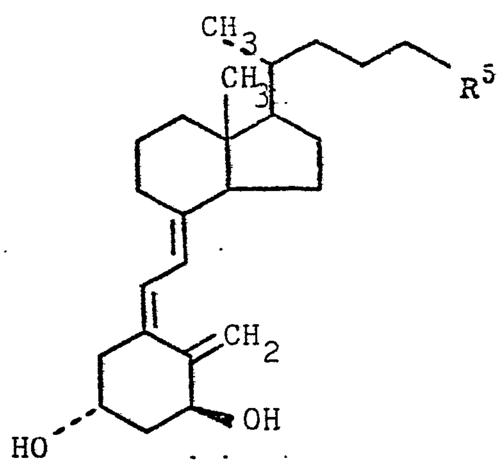

XI

worin $R^5$ eine Gruppe der Formel

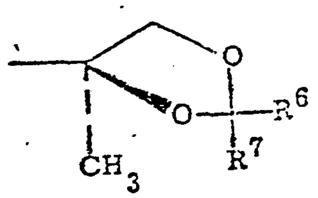

oder

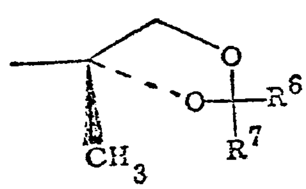

und $R^6$ und $R^7$ Niederalkyl oder zusammen Niederalkylen sind.

28. [1α,3β,5Z,7E,25S]-9,10-Secocholesta-5,7,10(19)-trien-1,3,25,26-tetrol-25,26-(1-methyläthyliden)-acetal.

29. [1α,3β,5Z,7E,25R]-9,10-Secocholesta-5,7,10(19)-trien-1,3,25,26-tetrol-25,26-(1-methyläthyliden)-acetal.

***

Patentanspruch für Oesterreich

Verfahren zur Herstellung des 1α,25,26-Trihydroxycholecalciferols in Form des C-25 R- or S-Epimeren oder in Form von Gemischen davon, dadurch gekennzeichnet, dass man

a) eine Verbindung der Formel

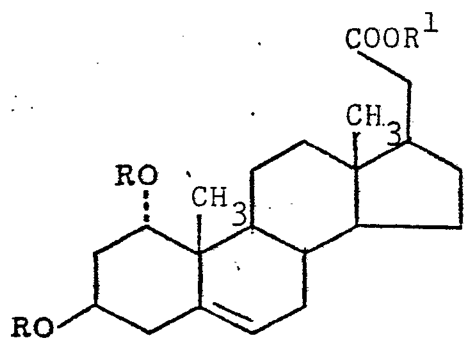

I

worin $R^1$ Niederalkyl, Phenyl oder substituiertes Phenyl und R eine Gruppe der Formel

$$-C(R^2,R^3,OR^4)$$

$R^2$ Wasserstoff oder Niederalkyl und $R^3$ und $R^4$ Niederalkyl oder zusammen $C_{3-6}$-Alkylen sind,

mit einem Lithium-, Natrium-, Kalium-, Magnesium- oder Zinkorganometallischen Reagenzien umsetzt,

b) das Produkt der Stufe a) der Formel

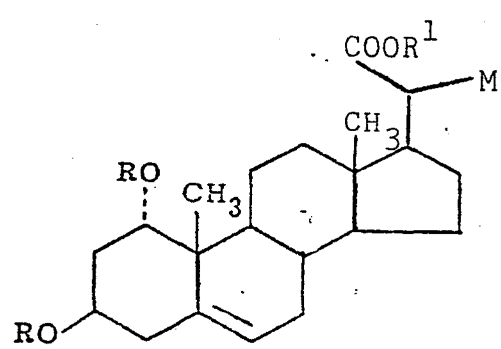

II

worin R und $R^1$ die obige Bedeutung haben und M Lithium, Natrium, Kalium, Magnesium/2 oder Zink/2 sind,

mit einer Verbindung der Formel

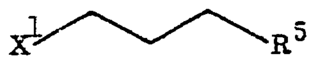

III

worin $X^1$ Jod, Brom, Chlor, Niederalkylsulfonyloxy, Phenylsulfonyloxy oder substituiertes Phenylsulfonyloxy und $R^5$ eine Gruppe der Formel

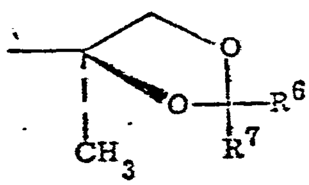

oder

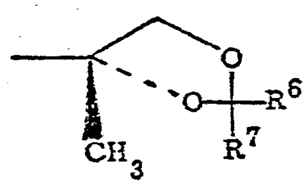

$R^6$ und $R^7$ Niederalkyl oder zusammen Niederalkylen sind,

c) den Ester von Stufe b) der Formel

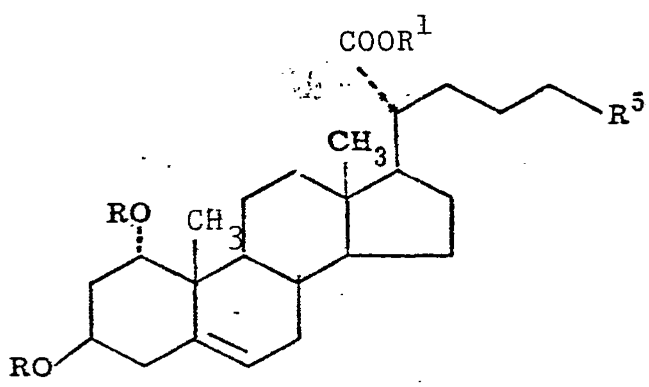

IV

worin R, $R^1$ und $R^5$ die obige Bedeutung haben, reduziert,

d) in dem Produkt der Stufe c) und der Formel

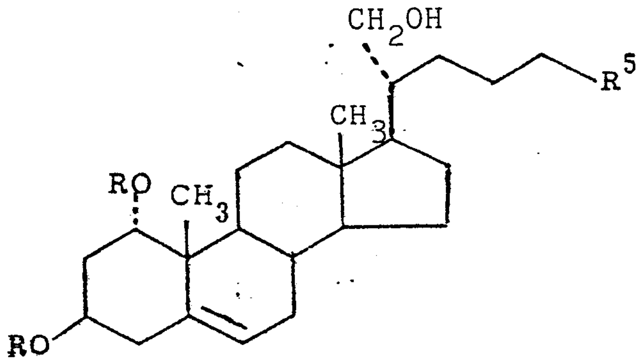

V

worin R und $R^5$ die obige Bedeutung haben, die Hydroxygruppe durch eine Gruppe $X^1$, worin $X^1$ obige Bedeutung hat, ersetzt,

e) das Produkt von Stufe d) und der Formel

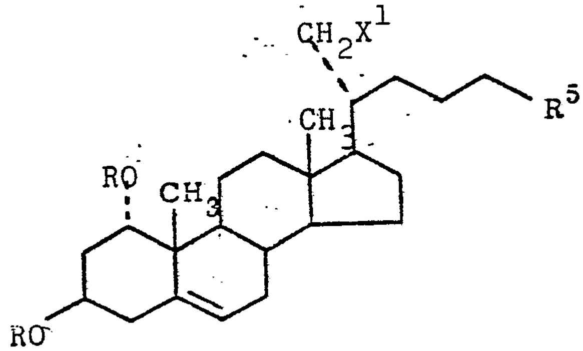

VI

worin R, $R^5$ und $X^1$ die obige Bedeutung haben, mit einem Reduktionsmittel umsetzt,

f) das Produkt der Stufe e) und der Formel

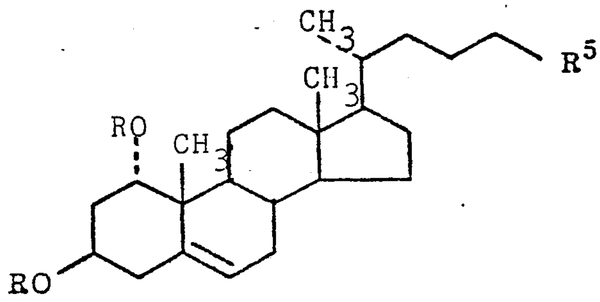

VII-A

worin R und $R^5$ die obige Bedeutung haben, mit einer starken Säure in einem Gemisch eines protischen Lösungsmittels und eines Ketallösungsmittels umsetzt,

g) das Produkt der Stufe f) und der Formel

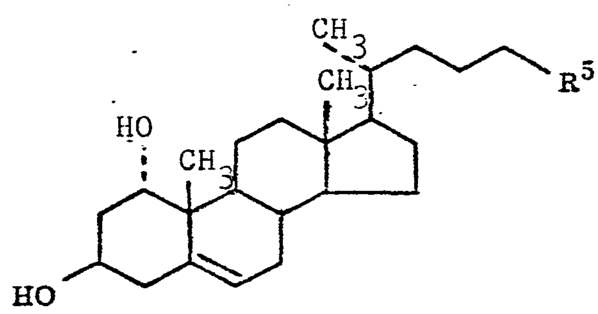

VII-B

worin $R^5$ obige Bedeutung hat, mit einem Niederalkanoylierungsmittel umsetzt,

h) das Produkt der Stufe g) und der Formel

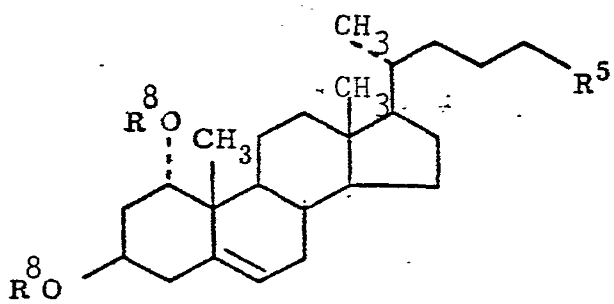

VII-C

worin $R^5$ die obige Bedeutung hat und $R^8$ Niederalkanoyl ist, in 7-Stellung allylisch halogeniert,

i) das Produkt der Stufe h) und der Formel

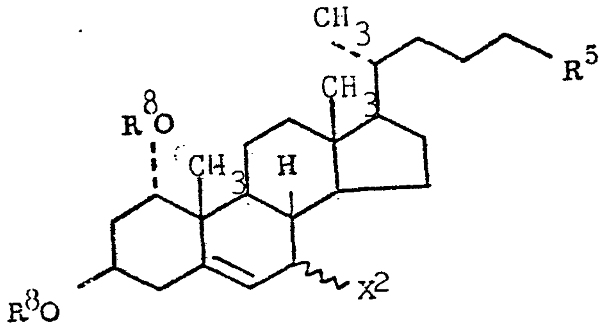

VIII

worin $R^5$ und $R^8$ die obige Bedeutung haben und $X^2$ Brom, Chlor oder Jod ist,

dehydrohalogeniert,

j) das Produkt der Stufe i) und der Formel

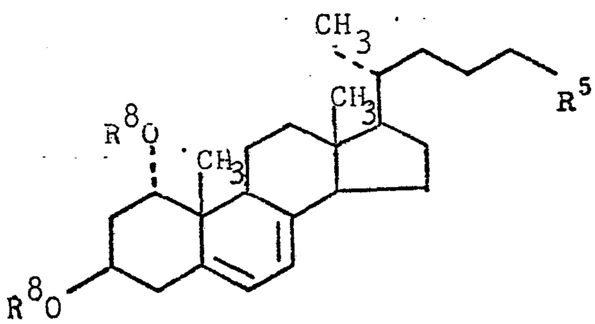

IX

worin $R^5$ und $R^8$ obige Bedeutung haben,

bestrahlt,

k) das Produkt der Stufe j) und der Formel

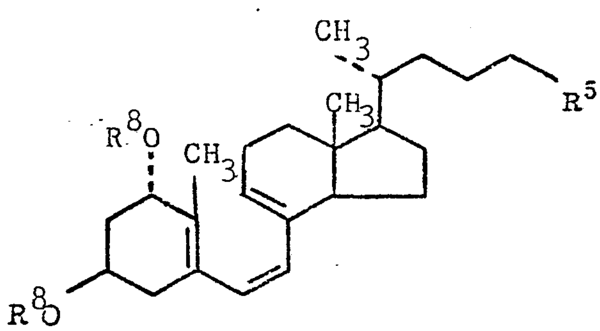

X-A

worin $R^5$ und $R^8$ obige Bedeutung haben, verseift,

l) das Produkt der Stufe k) und der Formel

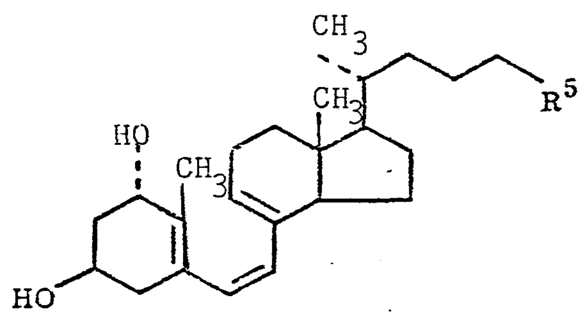

X-B

worin $R^5$ obige Bedeutung hat, thermisch isomerisiert und

m) das Produkt der Stufe l) und der Formel

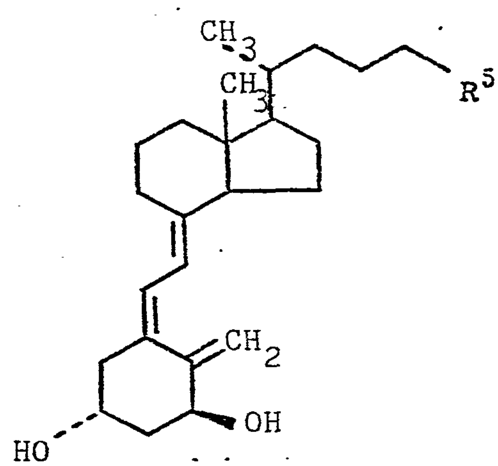

XI

worin $R^5$ obige Bedeutung hat, mit einer Säure in einem protischen Lösungsmittel behandelt.